# FASCICULE DE BREVET EUROPEEN

(11) **EP 2 831 227 B1**
(45) Date de publication et mention de la délivrance du brevet: **05.09.2018**
(21) Numéro de dépôt: 13712789.0
(22) Date de dépôt: 27.03.2013
(51) Int. Cl.: C12N 9/54, C07C 2/00, C12P 7/56

(54) **MICROORGANISME RECOMBINANT**
REKOMBINANTER MIKROORGANISMUS
RECOMBINANT MICROORGANISM

(30) Priorité: 27.03.2012 FR 1252733
(43) Date de publication de la demande: 04.02.2015
(73) Titulaire: Carbios, 63360 Saint-Beauzire (FR)
(72) Inventeur: BOISART, Cédric, F-63360 Gerzat (FR)
(74) Mandataire: Lebrette, Camille
(86) Numéro de dépôt international: PCT/EP2013/056583
(87) Numéro de publication internationale: WO 2013/144239

(56) Documents cités:
- MATSUDA EMIKO ET AL: "Gene cloning and molecular characterization of an extracellular poly(L-lactic acid) depolymerase from Amycolatopsis sp strain K104-1", JOURNAL OF BACTERIOLOGY, AMERICAN SOCIETY FOR MICROBIOLOGY, WASHINGTON, DC; US, vol. 187, no. 21, novembre 2005 (2005-11), pages 7333-7340, XP002616534, ISSN: 0021-9193, DOI: 10.1128/JB.187.21.7333-7340.2005
- DATABASE WPI Week 200950 Thomson Scientific, London, GB; AN 2009-K99963 XP002690934, -& CN 101 457 218 A (UNIV SHANDONG) 17 juin 2009 (2009-06-17)
- DATABASE WPI Week 200837 Thomson Scientific, London, GB; AN 2008-F66138 XP002690935, -& JP 2007 319092 A (TOYO SEIKAN KAISHA LTD) 13 décembre 2007 (2007-12-13)
- ZHAN-YONG WANG ET AL: "Gene Cloning and Characterization of a Poly(-Lactic Acid) Depolymerase fromsp. Strain DS04-T", JOURNAL OF POLYMERS AND THE ENVIRONMENT ; FORMERLY: 'JOURNAL OF ENVIRONMENTAL POLYMER DEGRADATION', KLUWER ACADEMIC PUBLISHERS-PLENUM PUBLISHERS, NE, vol. 19, no. 4, 28 août 2011 (2011-08-28), pages 827-833, XP019983218, ISSN: 1572-8900, DOI: 10.1007/S10924-011-0346-9
- AKUTSU-SHIGENO YUKIE ET AL: "Cloning and sequencing of a poly(DL-lactic acid) depolymerase gene from Paenibacillus amylolyticus strain TB-13 and its functional expression in Escherichia coli", APPLIED AND ENVIRONMENTAL MICROBIOLOGY, AMERICAN SOCIETY FOR MICROBIOLOGY, US, vol. 69, no. 5, mai 2003 (2003-05), pages 2498-2504, XP002616535, ISSN: 0099-2240, DOI: 10.1128/AEM.69.5.2498-2504.2003
- PETROV K ET AL: "l(+)-Lactic acid production from starch by a novel amylolytic Lactococcus lactis subsp. lactis B84", FOOD MICROBIOLOGY, ACADEMIC PRESS LTD, LONDON, GB, vol. 25, no. 4, juin 2008 (2008-06), pages 550-557, XP022655100, ISSN: 0740-0020, DOI: 10.1016/J.FM.2008.02.005 [extrait le 2008-02-29]

## Description

### Domaine de l'invention

L'invention concerne un microorganisme recombinant pour la production de monomères hydrocarbonés utiles à la synthèse de polymères. Plus précisément, l'invention porte sur un microorganisme génétiquement modifié de manière à produire de tels monomères à partir d'une source de carbone mais également par le biais de la dépolymérisation de polymères présents dans le milieu de culture. L'invention concerne également un procédé de production de monomères utilisant de tels microorganismes recombinants, ainsi qu'un procédé de synthèse de polymères à partir des monomères produits.

### Etat de la technique

Les polymères sont utilisés dans un grand nombre de domaines techniques, notamment sous forme de matière plastique, depuis les emballages alimentaires jusqu'au domaine médical, en passant par l'habillement, l'industrie automobile, etc. Par exemple les polyamides, et plus particulièrement les nylons, sont utilisés aussi bien pour la fabrication de semelles de chaussures que sous forme de microfilaments pour les sutures chirurgicales. De même, certains polyesters (par exemple le polyéthylène téréphthalate - PET, l'acide polylactique - PLA, etc.) sont utilisés dans la confection de vêtements et d'emballages, mais également sous forme de résine thermodurcissable pour la fabrication de pièces automobiles ou autres.

Jusqu'à récemment, les polymères utilisés dans la plupart des industries étaient issus d'énergies fossiles non renouvelables, telles que le pétrole. Cependant, les difficultés associées au recyclage de ces matériaux polymères et la pénurie croissante en énergie fossile non renouvelable rendent leur emploi de plus en plus critiquable.

Aussi, depuis quelques années, la fabrication de polymères à partir de matières premières renouvelables ou biomasse s'est développée. L'acide polylactique (PLA), par exemple, est un polymère biodégradable produit à partir d'acide lactique et qui présente des propriétés mécaniques comparables à celles de polymères issus de l'industrie pétrochimique. Des microorganismes ont été isolés et/ou développés pour leur capacité à produire des monomères hydrocarbonés et/ou des polymères à partir d'une source de carbone renouvelable adaptée.

Par exemple, différents microorganismes ont été développés pour produire de l'acide lactique à partir de sources de carbone comme l'amidon, les pentoses, etc. Notamment, des levures recombinantes, exprimant une lactate déshydrogénase, ont été produites pour synthétiser de l'acide lactique dans un milieu de culture approprié (WO03102152).

De même, des levures ont été génétiquement modifiées pour exprimer une fumarate réductase et produire de l'acide succinique à partir d'acide fumarique (WO2009065778).

Le plus souvent, ces microorganismes, recombinants ou non, doivent être cultivés dans un milieu de culture approprié comprenant des substrats onéreux (glucose, saccharose, etc.). Pour améliorer les performances de production de ces microorganismes, il est en outre nécessaire d'optimiser les conditions de culture (température, agitation, nature de la source de carbone etc.), ce qui tend encore à augmenter les coûts de production.

Afin de réduire les coûts de production, on a cherché à produire ces métabolites directement à partir de biomasse non directement valorisable, comme le jus de betterave (WO2008000699), le jus de dattes (Boudjelal et al. - Rev. Energ. Ren,: Production et Valorisation-Biomasse (2001) 41-46), etc. Cependant, les traitements préalables de la biomasse, pour y favoriser la croissance des microorganismes, sont complexes et longs, ce qui ne permet pas de réduire les coûts de production de manière satisfaisante.

Par ailleurs, le titre de production de monomères hydrocarbonés par des microorganismes dépasse difficilement les 30g/litre, et ce dans des conditions de culture optimales.

Aussi, les titres de production de monomères hydrocarbonés et/ou des polymères associés par des microorganismes restent encore relativement faibles et les coûts associés trop élevés pour être une réelle alternative au polyéthylène (PE) et autres polymères pétrochimiques. Bien que l'idée de produire des polymères à partir de ressources renouvelables séduise, il n'existe pas actuellement de système de production par fermentation qui pallie les problèmes de coût et/ou rendement et qui permette de répondre de manière satisfaisante à la demande.

### Résumé de l'invention

L'invention a pour objet de résoudre au moins partiellement au moins un des problèmes énoncés ci-dessus, en proposant un microorganisme recombinant susceptible de produire des quantités importantes de monomères d'acide lactique, utiles à la synthèse de polymères.

Pour cela l'invention propose un microorganisme, et notamment une bactérie, une levure ou un champignon, génétiquement modifié pour pouvoir synthétiser un ou plusieurs monomère(s) d'acide lactique par fermentation d'une source de carbone et dépolymériser, simultanément ou non, de l'acide polylactique présent dans le milieu de culture de manière à en libérer les monomères qui le composent. Les monomères ainsi produits par un même microorganisme proviennent de deux sources différentes et indépendantes. En outre, l'utilisation d'un tel microorganisme de l'invention permet de recycler les polymères et de résoudre ainsi les problèmes d'accumulation des déchets plastiques. En effet, les polymères susceptibles d'être dépolymérisés par le microorganisme selon l'invention peuvent aussi bien être intégrés dans un produit manufacturé en matière plastique, qu'être directement disponibles dans le milieu de culture en solution ou autre. Avantageusement, le microorganisme selon l'invention est apte à dépolymériser des polymères de haut poids moléculaire pas seulement en des oligomères de plus faible poids moléculaire, mais jusqu'à la forme synthon, ou monomère.

L'invention a donc pour objet un microorganisme génétiquement modifié de manière à
i) synthétiser un monomère d'acide lactique par fermentation d'une source de carbone, et ii) dépolymériser de l'acide polylactique (PLA), dans lequel ledit microorganisme exprime au moins une enzyme pour dépolymériser le PLA choisie parmi une protéinase K, une lipase et une PLA dépolymérase, et/ou une lactate déshydrogénase pour la synthèse d'acide lactique. L'invention a également pour objet un procédé de production d'un monomère d'acide lactique comprenant les étapes de
   - mise en contact d'un microorganisme selon l'invention avec une source de carbone et avec du PLA susceptible d'être dépolymérisé par ledit microorganisme, et optionnellement
   - récupération du monomère hydrocarboné produit.

L'invention vise également une coculture de microorganismes comprenant au moins un premier microorganisme selon l'invention et au moins un second microorganisme, éventuellement génétiquement modifié, apte à synthétiser un polymère constitué au moins des monomères produits par le premier microorganisme.

L'invention concerne également un procédé de synthèse de polymères utilisant une coculture de microorganismes selon l'invention, comprenant les étapes de
- mise en contact de la coculture de microorganismes avec une source de carbone et avec un polymère susceptible d'être dégradé par le premier microorganisme, et optionnellement
- récupération du polymère produit par le second microorganisme.

### Description des figures

- Figure 1 :: Dégradation du PLA en acide lactique en présence de quatre niveaux de concentration de protéinase K ( 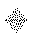 1.25 g/L, 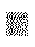 2.5 g/L, ▲ 3.75 g/L, 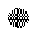 5 g/L).
- Figure 2 :: Evolution des taux de croissance (A) et de la biomasse (B) de *L. lactis IL 1403* dans du MCD sans autre rajout ( 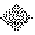 ), avec 20 g/L de PLA et 3.75 g/L de protéinase K (■) ou avec 20 g/L de PLA ( 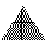 ).
- Figure 3 :: Evolution des concentrations en glucose (A) et des vitesses spécifiques de consommation du glucose (B) de *L. lactis IL 1403* dans du MCD sans autre rajout (◆), avec 20 g/L de PLA et 3.75 g/L de protéinase K (■) ou avec 20 g/L de PLA (▲).
- Figure 4 :: Evolution des concentrations en lactate (A) de *L. lactis IL 1403* dans du MCD sans autre rajout (◆), avec 20 g/L de PLA et 3.5 g/L de protéinase K (■) ou avec 20 g/L de PLA (▲) et calcul du différentiel en lactate (B) entre la condition sans PLA et les conditions avec PLA.
- Figure 5 :: Evolution des vitesses spécifiques de production du lactate de *L. lactis IL 1403* dans du MCD sans autre rajout (◆), avec 20 g/L de PLA et 3.75 g/L de protéinase K (■) ou avec 20 g/L de PLA (▲).

### Description détaillée

Dans le contexte de l'invention, on entend par microorganisme tout organisme unicellulaire eucaryote, tel que les levures, les microalgues et les champignons, ou procaryote, tel que les bactéries.

De préférence, le microorganisme est un microorganisme d'un genre choisi parmi *Aspergillus, Cupriavidus, Clostridium, Corynebacterium, Escherichia, Pseudomonas, Yarrowia, Aeromonas, Candida, Burkholderia, Thermobifida, Fusarium, Pichia, Saccharomyces* et *Bacillus.* De préférence, le microorganisme est choisi parmi *Aspergillus Niger, Cupriavidus necator, Clostridium acetobutylicum, Corynebacterium glutamicum, Escherichia coli, Pseudomonas aeruginosa, Pseudomonas putida, Pseudomonas entomophila, Pseudomonas oleovorans, Yarrowia lipolytica, Aeromonas hydrophila, Candida tropicalis, Candida antartica, Burkholderia xenovorans, Burkholderia mallei, Burkholderia pseudomallei, Thermobifida fusca, Fusarium solani, Pichia pastoris, Saccharomyces Cerevisiae, Bacillus subtilis* et *Bacillus megaterium.*

Par microorganisme « génétiquement modifié », on entend que le génome du microorganisme a été modifié de telle manière que ledit microorganisme présente les deux voies de synthèse et de dépolymérisation. Le microorganisme peut par exemple être un microorganisme apte naturellement à synthétiser un monomère d'intérêt à partir d'une source de carbone et qui est génétiquement modifié pour exprimer la ou les enzymes nécessaires à la dépolymérisation souhaitée, ou inversement. Le microorganisme peut autrement ne présenter naturellement aucune des voies de synthèse et de dépolymérisation recherchées, et être génétiquement modifié pour ces deux voies. Le microorganisme peut également présenter naturellement l'une et/ou l'autre de ces voies, et être génétiquement modifié pour favoriser et/ou augmenter l'expression des gènes impliqués. Selon l'invention, le génome du microorganisme est modifié de manière à intégrer au moins une séquence nucléique codant au moins une enzyme intervenant dans la voie de biosynthèse d'un monomère hydrocarboné et/ou dans la dépolymérisation d'un polymère, ou codant un fragment biologiquement actif de celle-ci. Ladite séquence nucléique peut avoir été introduite dans le génome dudit microorganisme ou d'un de ses ascendants, par le biais de toute méthode de clonage moléculaire adaptée. Dans le contexte de l'invention, le génome du microorganisme s'entend de l'ensemble du matériel génétique contenu dans ledit microorganisme, y compris le matériel génétique dans des plasmides, épisomes, chromosomes synthétiques, etc. La séquence nucléique introduite peut être une séquence hétérologue, c'est-à-dire qui n'existe pas à l'état naturel dans ledit microorganisme, ou une séquence homologue. Avantageusement, on introduit dans le génome du microorganisme une unité transcriptionelle comportant la séquence nucléique d'intérêt, placée sous le contrôle d'un ou plusieurs promoteur(s).

La source de carbone utilisée par le microorganisme selon l'invention comprend de préférence des sucres raffinés, comme le glucose, galactose, xylose, saccharose, etc., mais également toute matière organique d'origine végétale ou animale, ou biomasse, susceptible d'être dégradée par le microorganisme pour produire des sucres fermentescibles. Ainsi, la source de carbone peut comporter de la biomasse hémicellulosique, cellulosique, lignocellulosique, etc., par exemple de la mélasse et/ou de la bagasse de canne à sucre, les jus ou eaux pauvres de sucrerie, les eaux mères de cristallisation des sucres, etc. La source de carbone peut également comporter du glycérol ou des huiles (triglycérides). Dans tous les cas, la source de carbone ne comprend pas le monomère produit par dépolymérisation du polymère selon l'invention.

Le caractère simultané de la fermentation et la dépolymérisation dans la présente invention est défini par le fait que le microorganisme est mis en contact en même temps (ou dans un laps de temps très court, par exemple de l'ordre de 15 minutes maximum) avec la source de carbone et avec le polymère, dans les mêmes conditions physico-chimiques (par exemple température, pH et/ou milieu de culture). Ainsi, le monomère hydrocarboné produit par les deux voies (fermentation et dépolymérisation) peut être récupéré simultanément dans le milieu de culture.

Par monomère hydrocarboné, on entend l'acide lactique.

Par oligomère, on entend une succession de monomères selon l'invention comportant plus d'un monomère, et moins de monomères que le polymère fourni au microorganisme.

L'invention vise la dépolymérisation de l'acide polylactique (PLA) et la synthèse d'acide lactique

Selon l'invention, un même microorganisme est apte à synthétiser au moins un monomère d'acide lactique et à dépolymériser de l'acide polylactique. Selon l'invention, le microorganisme est apte à dépolymériser ledit polymère de manière à en récupérer le monomère d'intérêt.

Dans un exemple particulier de réalisation de l'invention, le microorganisme est également génétiquement modifié pour atténuer au moins une voie de dégradation du monomère d'acide lactique qu'il est apte à synthétiser.

En effet, dans certains cas, le microorganisme selon l'invention peut être naturellement apte à utiliser le monomère qu'il synthétise comme source de carbone pour sa croissance. Or, ces monomères peuvent présenter un intérêt industriel particulier, notamment pour la synthèse ultérieure de polymères, et il peut être intéressant d'éviter leur dégradation. Pour cela, selon l'invention il est possible de déléter tout ou partie des gènes codant la ou les enzymes impliquées dans la dégradation du monomère d'intérêt, par toute technique de recombinaison/délétion appropriée. Il est également possible d'atténuer l'expression de tout ou partie des gènes impliqués dans la dégradation du monomère d'intérêt. Pour cela, il est par exemple possible d'introduire une ou plusieurs séquences nucléiques codant des régulateurs, tels que des facteurs de transcription, des ARN interférents, de siARN etc., susceptibles d'atténuer l'expression de ces gènes. De même, il est possible de modifier la séquence promotrice d'au moins un des gènes impliqués dans la dégradation du monomère d'intérêt, de manière à en atténuer/empêcher la transcription.

Afin d'éviter la dégradation des monomères synthétisés, il est également possible, à la place ou en complément des modifications génétiques ci-dessus, d'enrichir régulièrement le milieu de culture du microorganisme en substrats facilement assimilables par le microorganisme, afin qu'il utilise préférentiellement ces substrats plutôt que le monomère synthétisé.

Le microorganisme selon l'invention peut également être génétiquement modifié de manière à pouvoir synthétiser un polymère constitué, en tout ou partie, du monomère qu'il est apte à synthétiser. Par exemple, le microorganisme peut être génétiquement modifié de manière à intégrer et exprimer une séquence nucléique codant une polymère synthase particulière. Ainsi, un même microorganisme peut, selon l'invention, produire un monomère par le biais d'une fermentation et d'une dépolymérisation ciblée d'un polymère à recycler/dégrader, et produire un nouveau polymère en utilisant les monomères produits. Dans le cas où le polymère d'intérêt est un hétéropolymère, il est possible d'enrichir le milieu de culture du ou des autres monomères constitutifs dudit polymère.

Ainsi, dans un premier exemple de réalisation, le microorganisme selon l'invention est génétiquement modifié pour dépolymériser le PLA et ainsi obtenir l'acide lactique, et concomitamment synthétiser de l'acide lactique à partir d'une source de carbone.

L'enzyme pour dépolymériser le PLA est avantageusement choisie parmi une serine protéase, une lipase et une PLA dépolymérase. De préférence, l'enzyme pour dépolymériser le PLA est choisie parmi la protéinase K de *Tritirachium album,* la lipase A *d'Aspergillus niger,* la lipase AY de *Candida rugosa,* la lipase F de *Rhizopus oryzae,* la lipase PS de *Burkholderia sp.* ou la lipase cutinase-like enzyme de la souche S-2 de *Cryptococcus* sp. De même, l'enzyme pour la synthèse d'acide lactique est avantageusement une lactate déshydrogénase. Dans un exemple particulier, le microorganisme est un microorganisme lactique, par exemple une bactérie lactique, exprimant naturellement, c'est-à-dire à l'état sauvage, une lactate déshydrogénase.

L'invention a également pour objet un procédé de production d'un monomère d'acide lactique comprenant les étapes de
- mise en contact d'un microorganisme selon l'invention avec une source de carbone et avec du PLA susceptible d'être dépolymérisé par ledit microorganisme, et optionnellement
- récupération du monomère d'acide lactique produit.

Le monomère d'acide lactique produit tant par fermentation que par dépolymérisation se retrouve avantageusement dans le milieu de culture. Il est alors possible de traiter le milieu de culture enrichi en ces monomères, par toute technique connue, de manière à les isoler et/ou les purifier.

Il est possible de produire toutes sortes de monomères, en adaptant simplement le microorganisme utilisé et les modifications génétiques introduites. Le choix du microorganisme ainsi que les modifications génétiques à apporter sont à la portée de l'homme du métier, au vu de l'enseignement de la présente demande.

Les conditions de la mise en contact du microorganisme avec la source de carbone et le polymère, telles que la température, le pH, etc., peuvent être adaptées par l'homme du métier pour optimiser/favoriser à la fois la production du monomère par dépolymérisation du polymère et la production du monomère par fermentation à partir de la source de carbone.

L'invention propose également une coculture, par exemple dans un fermenteur, d'au moins deux microorganismes, dont l'un est un microorganisme génétiquement modifié selon l'invention, le second microorganisme étant lui apte à synthétiser un polymère constitué au moins du monomère produit par le microorganisme selon l'invention.

Ainsi, le monomère produit par le microorganisme selon l'invention et excrété dans le milieu de culture est utilisé par le second microorganisme, pour produire un polymère d'intérêt, qui pourra être par la suite exploité dans tout genre d'industrie. Le procédé selon l'invention permet ainsi un recyclage complet du PLA, depuis leur dépolymérisation en monomères à nouveau exploitables, jusqu'à leur réutilisation sous forme de nouveaux polymères susceptibles d'être utilisés sous forme de plastiques pour de nouvelles applications.

Ainsi par exemple, le microorganisme selon l'invention est une bactérie lactique génétiquement modifiée qui exprime au moins une lactate déshydrogénase et au moins une enzyme susceptible de dépolymériser le PLA choisie parmi les PLA dépolymérases, les protéases et les lipases. Cette bactérie lactique peut être co-cultivée avec un autre microorganisme, éventuellement génétiquement modifié, apte à synthétiser du PLA. Avantageusement, les deux microorganismes co-cultivés sont choisis de manière à ne pas entrer en compétition pour la source de carbone, de préférence les sucres, présente dans le milieu de culture.Dans certains modes de réalisation de la présente invention, le terme « comprendre » peut signifier « consister essentiellement en », ou « consister en ».

### Exemples

L'invention sera mieux comprise à la lecture des exemples ci-dessous, donnés à titre illustratif et nullement limitatif de l'invention.

### Exemple 1 : Bactérie lactique recombinante exprimant une PLA dépolymérase

### Matériel & Méthode

Les techniques de clonage mises en oeuvre pour la préparation du plasmide recombinant sont conformes aux techniques décrites dans la publication de Sambrook et al. de 1989 («Molecular cloning : a laboratory manual. Cold spring Harbor Laboratory Press, Cold spring Harbor, NY. »*).*

Le gène pld *d'Amycolatopsis sp. K104-1* de séquence SEQ ID N°1 (Nakamura et al. 2001 - Appl. Environ. Microbiol. 67 :345-353) est introduit par recombinaison homologue dans le plasmide pNZ8048 (Kuipers et al. 1998 - J.Biotechnol. 64 :15-21). Le plasmide recombinant intégrant le gène pld est appelé « pNZ-pld ».

La souche sauvage *Lactococcus lactis MG1363* est transformée par le plasmide pNZ-pld introduit dans la cellule par électroporation conformément au procédé décrit dans Ho et al. de 1995 («Transformation of Lactococcus by electroporation » Methods Mol. Biol. 47 :195-199). La bactérie recombinante intégrant le plasmide pNZ-pld est appelée « MG1363-pNZ-pld ». Le témoin négatif « MG1363-pNZ8048 » correspond à la souche sauvage *Lactococcus lactis MG1363* transformée par le plasmide vide pNZ8048.

### Activité PLA dépolymérase

Les souches MG1363-pNZ-pld et MG1363-pNZ8048 sont cultivées en parallèle dans des fermenteurs de 2L en CDM («*Chemically Defined Medium*») à 30°C, en condition anaérobie.

Du glucose est ajouté dans le milieu de culture pour atteindre une concentration finale de 1% (poids/volume), et le pH est maintenu à 6.5 par ajout automatique de NaOH. Les cultures sont chacune divisées en deux sous-cultures, chaque lot de deux sous-cultures (MG1363-pNZ-pld-1/MG1363-pNZ8048-1 et MG1363-pNZ-pld-2/MG1363-pNZ8048-2) étant maintenu dans des conditions identiques.
Seul le second lot (MG1363-pNZ-pld-2/MG1363-pNZ8048-2) reçoit du PLA de masse moléculaire 220,000 Da (Shimadzu Co. -Kyoto, Japon), émulsionné dans le milieu de culture à 0.1% (poids/volume).

L'activité PLA dépolymérase est mesurée après 2 jours de culture, en appliquant la méthode décrite dans Nakamura et al. -2001 («Purification and characterization of an extracellular poly(L-lactic-acid) depolymerasefrom a soil isolate Amycolatopsis sp. Strain K104-1 » Appl. Environ. Microbiol. 67 :345-353).

Du surnageant de culture du premier lot est prélevé après deux jours de culture et analysé par spectrométrie de masse (Varian Inova 500) afin d'évaluer le taux d'acide lactique.
On observe pour les deux échantillons (MG1363-pNZ-pld-1 et MG1363-pNZ8048-1) un unique pic à 90 Da correspondant à l'acide lactique produit par les bactéries par fermentation. Ainsi, la souche recombinante MG1363-pNZ-pld est bien apte à produire de l'acide lactique par fermentation du glucose présent dans le milieu de culture.

En parallèle, du surnageant de culture du second lot est prélevé et analysé par spectrométrie de masse (Varian Inova 500) afin d'évaluer le taux d'oligomères d'acide lactique (essentiellement dimères et trimères) présents.
On observe pour les deux échantillons (MG1363-pNZ-pld-2 et MG1363-pNZ8048-2) un pic à 220,000 Da correspondant au PLA présent dans le milieu de culture et un pic à 90 Da correspondant à l'acide lactique. Par ailleurs, pour l'échantillon correspondant au milieu de culture de MG1363-pNZ-pld-2, on observe la présence de plusieurs pics de poids moléculaire intermédiaire, absents pour MG1363-pNZ8048-1. Ces pics intermédiaires correspondent à des oligomères d'acide lactique de tailles variables, ce qui confirme que du PLA du milieu de culture a bien été dégradé par la souche recombinante MG1363-pNZ-pld-2.

La souche recombinante MG1363-pNZ-pld, exprimant une PLA dépolymérase, est bien apte à produire de l'acide lactique par fermentation et à dépolymériser du PLA de haut poids moléculaire présent dans le milieu de culture.

### Exemple 2 : Evaluation de la production d'acide lactique

Les surnageants de culture de l'exemple 1 correspondant aux lots MG1363-pNZ-pld-1, MG1363-pNZ-pld-2, MG1363-pNZ8048-1 et MG1363-pNZ8048-2 sont analysés par HPLC afin de déterminer la quantité d'acide lactique libéré. Pour cela, une colonne Aminex HPX-87H, thermostatée à 50°C et éluée à 0,5 mL/min par une solution d'acide sulfurique à 5 mM est utilisée pour la quantification.

Cette analyse permet de démontrer que la quantité d'acide lactique dans le surnageant du lot MG1363-pNZ-pld-2 est supérieure à la quantité d'acide lactique des 3 autres conditions (MG1363-pNZ-pld-1, MG1363-pNZ8048-1 et MG1363-pNZ8048-2). De même, la présence d'un dimère d'acide lactique est observée uniquement dans le surnageant de culture du lot MG 1363-pNZ-pld-2.

Une analyse complémentaire sur une colonne C18 est réalisée selon la méthode décrite par Vu et al., 2005 («Oligomer distribution in concentrated lactic acid solutions », Fluid Phase Equilibria, 236,125-135). L'analyse du surnageant de culture du lot MG1363-pNZ-pld-2 confirme la présence d'oligomères d'acide lactique jusqu'à un degré de polymérisation de 6.

### Exemple 3 : Culture de Lactococcus lactis IL 1403 en présence de protéinase K.

L'exemple ci-dessous traite de la combinaison de la souche *Lactococcus lactis IL 1403* et de la protéinase K pour la production d'acide lactique. La protéinase K est la protéinase K de *Tritirachium album,* disponible chez EUROMEDEX sous la référence EU0090.

### 3.1. Choix de la souche et du milieu de culture

La souche utilisée est la souche *Lactococcus lactis IL 1403.* Cette souche a été cultivée sur milieu chimiquement défini (milieu MCD) additionné de 5 g/L de glucose et tamponné à pH 8. Un milieu riche (contenant des sources de peptones, de protéines ou d'oligopeptides) a été écarté afin de ne pas inclure dans le milieu de culture des substrats préférentiels à la protéinase K qui pourraient entrer en compétition avec le PLA que l'on souhaite dégrader.

**Tableau 1 : Composition du milieu chimiquement défini (MCD) à pH 8**

| **Composés** | **Concentration (g/L)** |
|---|---|
| glucose | 5 |
| sodium acetate | 1 |
| ammonium citrate | 0.6 |
| KH₂PO₄ | 0.9 |
| K₂HPO₄ | 31.2 |
| MgCl₂. 6H₂O | 0.2 |
| FeSO₄. 0H₂O | 0.011 |
| CaCl₂. 2H₂O | 0.05 |
| ZnSO₄. 7H₂O | 0.005 |
| COCl₂. 6H₂O | 0.0025 |
| Alanine | 0.24 |
| arginine | 0.12 |
| asparagine | 0.34 |
| glutamine | 0.51 |
| Glycine | 0.17 |
| histidine | 0.11 |
| lysine | 0.35 |
| methionine | 0.12 |
| Proline | 0.68 |
| Serine | 0.34 |
| thréonine | 0.23 |
| tryptophane | 0.05 |
| isoleucine | 0.2 |
| Leucine | 0.47 |
| Valine | 0.33 |
| phénylalanine | 0.28 |
| tyrosine | 0.29 |
| Adenine | 0.01 |
| Guanine | 0.01 |
| Uracile | 0.01 |
| xanthine | 0.01 |
| acide P-aminobenzoique | 0.01 |
| Biotine | 0.01 |
| cyano-cobalamine (B12) | 0.001 |
| acide folique | 0.001 |
| Inosine | 0.005 |
| Acide orotique | 0.005 |
| Ca panthotenate | 0.001 |
| pyridoxamine | 0.005 |
| pyridoxine (B6) | 0.002 |
| riboflavine (B2) | 0.001 |
| thiamine | 0.001 |
| Acide D,L-6,8-thioctique | 0.0025 |
| thymidine | 0.005 |
| cystéine | 0.17 |

### 3.2. Choix des conditions de culture

L'objectif a consisté à déterminer le taux de croissance de *L. lactis IL 1403* pour 4 valeurs de pH : 6.6, 7.0, 7.5 et 8.0. Les expérimentations ont été réalisées en tube contenant 15 mL de milieu chimiquement défini avec 5 g/L de glucose à 30 °C. Ces expérimentations ont été effectuées en condition d'anaérobiose et en condition d'aérobiose.

La croissance a été suivie par mesure de la densité optique à 580 nm pendant 24 h, et le taux de croissance moyen a pu être déterminé.

**Tableau 2 : Paramètres de croissance de L. lactis IL 1403 à différents pH en condition d'anaérobiose et d'aérobiose**

| | **anérobiose** | | **aérobiose** | |
|---|---|---|---|---|
| **IL1403** | µ_{moyen} (h⁻¹) | µ (%) | µ_{moyen} (h⁻¹) | µ (%) |
| 6.6 | 0.52 | 100 | 0.64 | 100 |
| 7.0 | 0.55 | 106 | 0.59 | 93 |
| 7.5 | 0.47 | 90 | 0.59 | 93 |
| 8.0 | 0.36 | 69 | 0.48 | 75 |

Si la condition à pH 6.6 correspond à la condition de référence pour *L. lactis IL 1403,* le taux de croissance maximal décroit faiblement à pH 7.5 mais de façon un peu plus importante à pH 8 (25-30 %). Il y a peu d'influence des conditions d'aération.

Ces résultats montrent que *L. lactis IL 1403* peut être cultivée à pH 8 avec peu d'influence sur son taux de croissance.

### 3.3. Choix des conditions de dépolymérisation du PLA

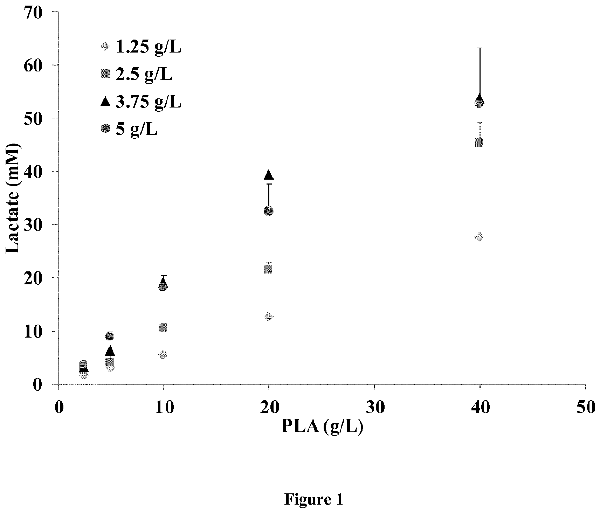

Une première étude a été réalisée afin de déterminer l'efficacité d'hydrolyse de la protéinase K à différentes concentrations (1.25, 2.5, 3.75 et 5 g/L) dans le milieu chimiquement défini (milieu de croissance de *L. lactis IL 1403*) à 30°C (température de croissance de la bactérie lactique) et à pH 8 (pH optimal de la protéinase K). La concentration en acide lactique libéré après 24 h d'hydrolyse a été mesurée par HPLC pour 5 concentrations de PLA initial : 2.5, 5, 10, 20 et 40 g/L et 4 concentrations de protéinase K : 1.25, 2.5, 3.75 et 5 g/L (Figure 1).

La figure 1 compare la dégradation du PLA en acide lactique en présence de 4 niveaux de concentrations de protéinase K.

Cette étude montre qu'au bout de 24 h entre 27 et 54 mM d'acide lactique peuvent être obtenus par hydrolyse du PLA par la protéinase K en MCD à un pH initial de 8.0. Le meilleur rendement d'hydrolyse est obtenu pour des concentrations de PLA et de protéinase K de 20 g/L et de 3.75 g/L respectivement.

### 3.4. Combinaison de la bactérie lactique et de la PLA dépolymérase

Les expérimentations ont été réalisées dans des fermenteurs 2 L (Biostat B plus, Sartorius ; 1 L de volume de travail) pilotés par ordinateur. Trois conditions de culture ont été testées à partir d'un milieu chimiquement défini contenant seulement 2.5 g/L de glucose. Le pH a été régulé à une valeur de 8.0 par ajout de KOH 10 N. La température était de 30°C. Les trois conditions sont décrites ci-dessous :
A. MCD + IL 1403
B. MCD + IL 1403 + PLA 20 g/L + Protéinase K (3.75 g/L)
C. MCD + IL 1403 + PLA 20 g/L

Le PLA a été autoclavé à sec directement dans les fermenteurs. Le milieu et la protéinase K ont été rajoutés stérilement.

Un suivi cinétique des cultures a été effectué : prélèvement toutes les demi-heures, mesure des densités optiques à 580 nm, congélation des surnageants de culture après centrifugation pour analyse ultérieure du substrat et co-produits par HPLC. La corrélation entre DO et poids sec pour la souche *L. lactis IL 1403* est connue : 1 g/L = 0.3 UDO.

### 3.4.1. Croissance

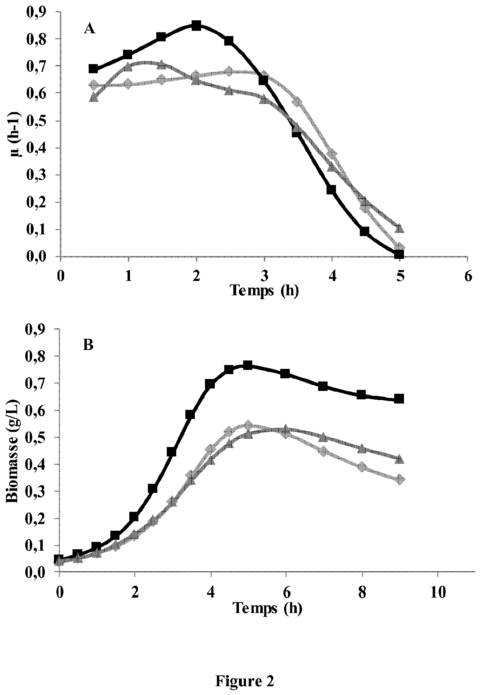

Les profils des taux de croissance de *L. lactis IL 1403* cultivée en l'absence et en présence de PLA sont similaires. Les µmax sont de 0.65 h-1 (sans PLA) et 0.70 h-1(avec PLA). En revanche, le profil du taux de croissance lorsque de la protéinase K est rajoutée dans le milieu, en supplément du PLA, est très différent. Le µmax est de 0.85 h-1 (Figure 2A). Cette observation est en corrélation avec le suivi de la biomasse (Figure 2B). L'ajout de PLA ne modifie pas la production de biomasse. Nous pouvons observer des profils pratiquement identiques et une biomasse maximale de 0.52 et 0.47 g/L, respectivement sans ou avec ajout du PLA. La présence de protéinase K permet d'atteindre une biomasse maximale nettement plus élevée soit 0.75 g/L. Cette augmentation de biomasse n'est pas liée à une interférence de l'hydrolyse du PLA par la protéinase K avec la DO.

Quelles que soient les conditions de milieu, l'arrêt de croissance est effectif après 4h30 de culture et correspond à la consommation totale du glucose.

### 3.4.2. Bilan catabolique

Le suivi dynamique des différentes conditions de culture de *L. lactis IL 1403* a été réalisé durant 9 heures, puis deux prélèvements finaux ont été réalisés à 15 heures, et à 24 heures.

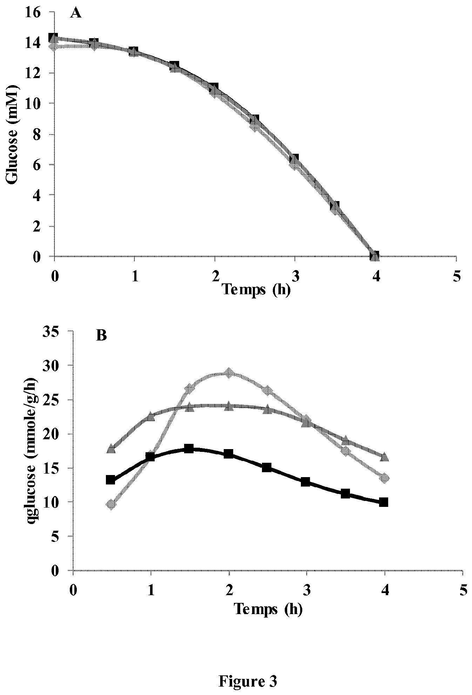

L'évolution des concentrations en glucose dans le milieu de culture est strictement similaire dans les trois conditions de culture. Le glucose est totalement consommé au bout de 4h de culture (Figure 3A). Quant aux vitesses spécifiques de consommation du glucose lors de la phase exponentielle de croissance (lh-3h), elles sont très proches en milieu sans PLA et avec PLA. Les valeurs maximales des vitesses spécifiques de consommation de glucose (qglucose) sont respectivement de 27 et 24 mmole/g/h. Lorsque la protéinase K est rajoutée dans le milieu, ce qglucose est seulement de 17 mmole/g/h. Cette différence de qglucose est en concordance avec une biomasse plus importance dans le fermenteur contenant de la protéinase K.

Le lactate est le produit de fermentation majoritaire. L'ajout de PLA et de protéinase K ne modifie en rien le caractère homolactique de la souche. Plus de 90% du flux de pyruvate conduisent à la production d'acide lactique, tandis que moins de 7% sont dirigés vers la production d'acide acétique. Les bilans carbones cataboliques sont équilibrés entre 89 et 95 %, en prenant en compte le CO2 théoriquement produit par le métabolisme. Les bilans stoechiométriques globaux en mM de carbone, calculés après 24 h de culture sont les suivants :
* **IL 1403**

| | |
|---|---|
| **0-24 h :** | 14.2 Glucose → 24.7 Lactate + 2.1 Acétate + 2.1 CO₂ |

*** IL 1403 avec PLA**

| | |
|---|---|
| **0-24 h :** | 14.3 Glucose → 23.8 Lactate + 1.5 Acétate + 1.5 CO₂ |

*** IL 1403 avec PLA et protéinase K**

| | |
|---|---|
| **0-24 h :** | 14 .3 Glucose → 23.8 Lactate + 1.5 Acétate + 1.5 CO₂ |

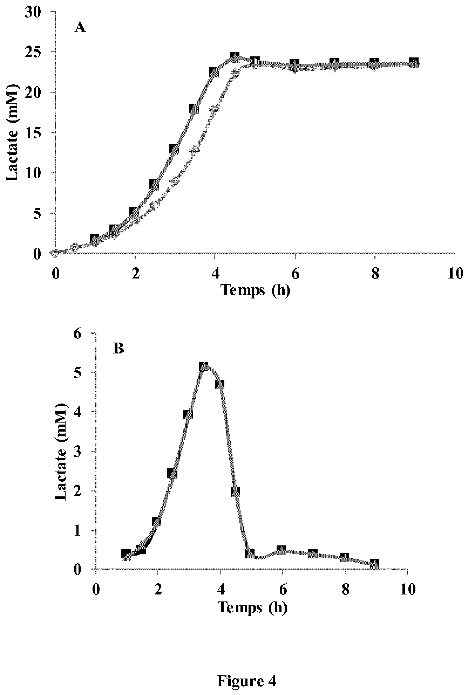

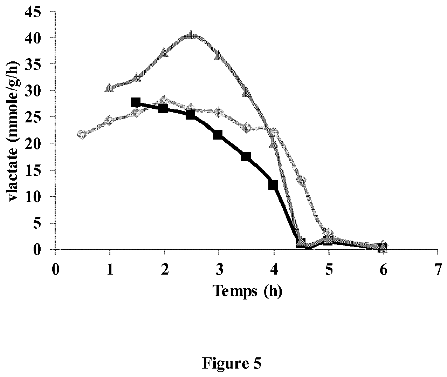

L'évolution des concentrations en lactate suit globalement le même profil dans les trois conditions de culture, avec cependant des nuances en début de culture lors de la présence de PLA avec ou sans protéinase K (Figure 4A). En effet, le PLA a été autoclavé, entrainant une possible fragilisation thermique ou une autohydrolyse. Cette différence de concentration (moins de 5 mM, cf Figure 4B) par rapport à la condition sans ajout de PLA n'est pas très significative. Elle est d'autre part rigoureusement identique en présence ou non de la protéinase K et ne peut donc en aucun cas être attribuée à l'hydrolyse du PLA par la protéinase K. Quant aux vitesses spécifiques de production de lactate (Figure 5), aucune différence majeure entre les trois conditions n'a été observée.

### 3.5. Conclusion

Dans les essais réalisés en fermenteur à pH régulé, la dégradation de PLA en acide lactique par la protéinase K n'a pas pu être détectée. La production de lactate peut être considérée comme provenant exclusivement de la consommation du glucose : 1.66 à 1.74 mmole de lactate produit par mmole de glucose, valeur classiquement rencontrée pour cette souche.

Ceci démontre que le simple ajout de la PLA-dépolymérase dans une culture de bactérie lactique ne permet pas une augmentation de l'acide lactique produit dans le procédé.

### Exemple 4 : souche recombinante pour la production de succinate

### 4.1. Construction d'une souche d'Escherichia coli exprimant une PBS-dépolymérase

Une souche *d'Escherichia coli* SBS 550 MG- PHL413 exprimant une PBS-dépolymérase (Cutinase-like enzyme (CLE) de *Cryptococcus sp. S*-2) est construite. La séquence du gène de la CLE est présentée dans SEQ ID N° 2, la séquence d'acides aminés correspondante est présentée dans SEQ ID N°3.

Pour cela, le gène de la CLE est amplifié par PCR à partir de l'ADN génomique de la souche *Cryptococcus sp. S-2* (Masaki et al., 2005 : Kazuo Masaki, Numbi Ramudu Kamini, Hiroko Ikeda, and Haruyuki Iefuji, "Cutinase-Like Enzyme from the Yeast Cryptococcus sp. Strain S-2 Hydrolyzes Polylactic Acid and Other Biodegradable Plastics", AEM, 71, 7548-7550).

Les amorces utilisées sont les suivantes :
- Amorce sens portant un site de restriction BamHI :
   GATCGGATCCGCCACGTCCAGCGCTTGTCCG (SEQ ID N°6)
- Amorce anti-sens portant un site de restriction XhoI :
   CCGGAACTCGAGGGCCGACCCGCCAAGCTTGTTG (SEQ ID N°7)

Parallèlement, le vecteur pET26b(+) (Novagen) est linéarisé par digestion par les enzymes de restriction BamHI et XhoI.

L'insert et le vecteur linéarisé sont purifiés sur gel à l'aide du QIAquick Gel Extraction kit (Qiagen). Une ligation est réalisée avec la ligase T4 (T4 DNA ligase, New England Biolabs). Le produit de ligation est transformé dans *E. coli DH5a* puis étalé sur milieu LB-Agar contenant 40 µg/mL de kanamycine. Après une nuit à 37°C, une colonie est prélevée et cultivée dans 5 mL de milieu LB contenant 40 µg/mL de kanamycine à 37°C pendant une nuit (16 à 18 h). Le vecteur amplifié est purifié à l'aide du kit QIAprep Spin Miniprep Kit (Qiagen) et vérifié par séquençage.

Le vecteur ainsi obtenu est appelé pET26b-CLE.

Le vecteur pET26b-CLE est transformé dans la souche *E. coli SBS 550 MG* - *PHL413* (Sanchez et al., 2005 : Ailen M. Sanchez, George N. Bennett, Ka-Yiu San, « Novel pathway engineering design of the anaerobic central metabolic pathway in Escherichia coli to increase succinate yield and productivity», Metabolic Engineering 7 (2005) 229-239). Un témoin négatif est construit par transformation du vecteur pET26b vide dans *E. coli SBS 550 MG - PHL413.* Les deux souches ainsi obtenues sont respectivement appelées *E. coli SBS 550 MG CLE et E. coli SBS 550 MG pET26b.*

### 4.2. Production d'acide succinique par culture de la souche E. coli SBS 550 MG -CLE

Les souches *E. coli SBS 550 MG -CLE et E. coli SBS 550 MG pET26b* sont cultivées en fermenteur de 2 L dans un milieu minéral additionné de glucose à 20 g/L et de PBS à 20 g/L (Bionolle 1001, Showa Highpolymer Corp. Ltd. (Tokyo, Japan) micronisé à 500 µm). La température est maintenue à 37°C, le pH à 6,5 par ajout de KOH. Une première phase de culture en conditions aérobies permet la production de biomasse. Lorsque la densité optique à 600 nm atteint 0,6, une solution d'IPTG est ajoutée dans chaque fermenteur de manière à obtenir une concentration finale de 1 mM et d'induire la production de la PBS-dépolymérase dans le fermenteur correspondant à la culture de la souche *E. coli SBS 550 MG -CLE.* La culture est poursuivie en condition anaérobie par injection continue de CO2 à 0,4 vvm jusqu'à consommation totale du glucose.

Des prélèvements du milieu de culture sont réalisés à intervalle régulier afin de mesurer la croissance bactérienne et d'analyser la composition du surnageant. La concentration en glucose ainsi que la production d'acide succinique est mesurée par HPLC sur une colonne Bio-rad HPX 87H. La détection est effectuée par réfractométrie.

Au cours de la fermentation, le glucose est bien consommé par chacune des souches et de l'acide succinique est produit. Le fermenteur correspondant à la culture de la souche *E. coli SBS 550 MG -CLE* montre une production plus importante d'acide succinique correspondant à la dépolymérisation du PBS par la CLE (PBS-dépolymérase) exprimée par la souche *E. coli SBS 550 MG -CLE.*

### Exemple 5 : souche recombinante pour la production de 1,3-propanediol

### 5.1. Construction d'une souche de Clostridium acetobutylicum exprimant une PTT-dépolymérase.

Le gène Btal correspondant à la cutinase TfH est amplifié à partir de l'ADN génomique issu de la souche *Thermobifida fusca DSM 43793.* La séquence du gène Bta1 de la cutinase TfH est présentée dans SEQ ID N° 4, la séquence d'acides aminés correspondante est présentée dans SEQ ID N°5. Parallèlement, le peptide signal PS-Ce148A de la cellulase de *Clostridium acetobutylicum ATCC 824* (Ce148A, Sabathé, F., A. Belaich, and P. Soucaille. 2002. Characterization of the cellulolytic complex (cellulosome) of Clostridium acetobutylicum. FEMS Microbiol. Lett. 217:15-22) est amplifié par PCR (Réaction en chaîne par polymérase).

Le vecteur d'expression pSOS952 (Perret, S., Casalot, L., Fierobe, H. P., Tardif, C., Sabathe, F., Belaich, J. P., and Belaich, A., 2004. "Production of heterologous and chimeric scaffoldins by Clostridium acetobutylicum ATCC 824". J. Bacteriol. 186, 253-257) est linéarisé par digestion avec les enzymes de restriction BamHI et Nar I.

Finalement, le clonage du gène btal précédé du peptide signal PS-Cel48A est cloné dans le vecteur linéarisé par utilisation du kit de clonage In-Fusion (In-Fusion® HD Cloning Kit, Clontech). Le vecteur ainsi obtenu est appelé pSOS952-btal.

Le vecteur pSOS952-bta1 est transformé dans *E. coli ER2275 (pAN1)* pour être méthylé par la méthyltransférase φ3T I selon la méthode décrite par Mermelstein et Papoutsakis, 1993 et Green et al., 1996. Après purification, le plasmide est transformé par électroporation dans la souche *Clostridium acetobutylicum DG1 (pSPD5)* (Gonzalez et al., 2005). La souche recombinante obtenue est appelée C. *acetobutylicum DG1-pSOS952-bta1.*

Une souche contrôle (témoin négatif) est construite par transformation du vecteur pSOS952 dans *Clostridium acetobutylicum DG1 (pSPD5).*

### 5.2. Production de 1,3-propanediol par culture de la souche C. acetobutylicum DG1-pSOS952-bta1

Une pré-culture des deux souches ainsi obtenues est réalisée à 35°C sur milieu synthétique contenant 10 g/L de glycérol comme source de carbone et en condition anaérobie (fiole sertie et sous atmosphère d'azote, Gonzalez et al., 2005). Chaque pré-culture est utilisée pour ensemencer un fermenteur de 250 mL de manière à avoir une absorbance à 600 nm égale à 0,05 à t0. Le milieu de culture est constitué du milieu synthétique décrit par Gonzalez et al., 2005 supplémenté par du glycérol à 50 g/L et du PTT (Polytriméthylène terephthalate, Sorona micronisé à 500 µm, DuPont) et maintenu en condition anaérobie par une atmosphère d'azote. Le pH du fermenteur est maintenu à 6,5 par ajout de NH4OH.

Des prélèvements du milieu de culture sont réalisés à intervalle régulier afin de mesurer la croissance bactérienne et d'analyser la composition du surnageant. La concentration en glycérol ainsi que la production de 1,3-propanediol est mesurée par HPLC sur une colonne Bio-rad HPX 97H. La détection est effectuée par réfractométrie.

Au cours de la fermentation, le glycérol est bien consommé par chacune des souches et du 1,3-propanediol et de l'acétate sont produits. Le fermenteur correspondant à la culture de la souche C. *acetobutylicum DG1-pSOS952-bta1* montre une production plus importante de 1,3 propanediol, ainsi qu'une production d'acide téréphtalique, ce qui correspond à la dépolymérisation du PTT par la PTT-dépolymérase sécrétée dans le milieu de culture.

## Revendications

1. Microorganisme génétiquement modifié de manière à
i) synthétiser un monomère d'acide lactique par fermentation d'une source de carbone, et
ii) dépolymériser de l'acide polylactique (PLA),
dans lequel ledit microorganisme exprime au moins une enzyme pour dépolymériser le PLA choisie parmi une protéinase K, une lipase et une PLA dépolymérase, et une lactate déshydrogénase pour la synthèse d'acide lactique.

2. Microorganisme selon la revendication 1, génétiquement modifié de manière à
iii) atténuer les voies de dégradation du monomère d'acide lactique qu'il est apte à synthétiser.

3. Microorganisme selon l'une des revendications 1 à 2, génétiquement modifié de manière à
iv) synthétiser du PLA constitué au moins du monomère d'acide lactique qu'il est apte à synthétiser.

4. Microorganisme selon l'une des revendications 1-3, selon lequel ledit microorganisme est un microorganisme lactique, préférentiellement une bactérie lactique.

5. Procédé de production d'un monomère d'acide lactique comprenant l'étape de
- mise en contact d'un microorganisme selon l'une des revendications 1 à 4, avec une source de carbone et avec du PLA susceptible d'être dépolymérisé par ledit microorganisme.

6. Procédé de production d'un monomère hydrocarboné selon la revendication 5, comprenant en outre l'étape de
- récupération du monomère d'acide lactique produit.

7. Coculture de microorganismes comprenant au moins un premier microorganisme selon l'une des revendications 1 à 4 et au moins un second microorganisme, éventuellement génétiquement modifié, apte à synthétiser un polymère constitué au moins des monomères d'acide lactique produits par le premier microorganisme.

8. Procédé de synthèse de polymères utilisant une coculture de microorganismes selon la revendication 7, comprenant l'étape de
- mise en contact de la coculture de microorganismes avec une source de carbone et avec un polymère susceptible d'être dégradé par le premier microorganisme.

9. Procédé de synthèse de polymères utilisant une coculture de microorganismes selon la revendication 8, comprenant en outre l'étape de
- récupération du PLA produit par le second microorganisme.

10. Procédé de synthèse selon la revendication 9, pour la production de PLA, selon lequel le premier microorganisme est un microorganisme selon l'une des revendications 1 à 4, et le second microorganisme est génétiquement modifié pour synthétiser du PLA à partir de monomères d'acide lactique.

## Patentansprüche

1. Mikroorganismus, der gentechnisch verändert ist, um
i) ein Milchsäure-Monomer durch Fermentation einer Kohlenstoffquelle zu synthetisieren, und
ii) Polymilchsäure (PLA) zu depolymerisieren,
wobei besagter Mikroorganismus wenigstens ein Enzym für die Depolymerisierung der PLA, ausgewählt aus Proteinase K, einer Lipase und einer PLA-Depolymerase, und eine Lactat-Dehydrogenase für die Synthese der Milchsäure exprimiert.

2. Mikroorganismus gemäß Anspruch 1, der gentechnisch verändert ist, um
iii) die Abbauwege des Milchsäure-Monomers einzuschränken, zu dessen Synthese er in der Lage ist.

3. Mikroorganismus gemäß einem der Ansprüche 1 bis 2, der gentechnisch verändert ist, um
iv) PLA zu synthetisieren, die wenigstens aus dem Milchsäure-Monomer besteht, zu dessen Synthese er in der Lage ist.

4. Mikroorganismus gemäß einem der Ansprüche 1 bis 3, wobei besagter Mikroorganismus ein Milchsäure-Mikroorganismus, vorzugsweise ein Milchsäurebakterium, ist.

5. Verfahren zur Produktion eines Milchsäure-Monomers, umfassend den Schritt:
- Inkontaktbringen eines Mikroorganismus gemäß einem der Ansprüche 1 bis 4 mit einer Kohlenstoffquelle und mit PLA, die von besagtem Mikroorganismus depolymerisiert werden kann.

6. Verfahren zur Produktion eines Kohlenhydrat-Monomers gemäß Anspruch 5, umfassend außerdem den Schritt:
- Wiedergewinnung des produzierten Milchsäure-Monomers.

7. Co-Kultur von Mikroorganismen, umfassend wenigstens einen ersten Mikroorganismus gemäß einem der Ansprüche 1 bis 4 und wenigstens einen zweiten Mikroorganismus, der gegebenenfalls gentechnisch verändert ist und in der Lage ist, ein Polymer zu synthetisieren, das wenigstens aus Milchsäure-Monomeren besteht, die von dem ersten Mikroorganismus produziert werden.

8. Verfahren zur Synthese von Polymeren unter Verwendung einer Co-Kultur von Mikroorganismen gemäß Anspruch 7, umfassend den Schritt:
- Inkontaktbringen der Co-Kultur von Mikroorganismen mit einer Kohlenstoffquelle und einem Polymer, das von dem ersten Mikroorganismus abgebaut werden kann.

9. Verfahren zur Synthese von Polymeren unter Verwendung einer Co-Kultur von Mikroorganismen gemäß Anspruch 8, umfassend außerdem den Schritt:
- Wiedergewinnung der von dem zweiten Mikroorganismus produzierten PLA.

10. Verfahren zur Synthese gemäß Anspruch 9 für die PLA-Produktion, wobei der erste Mikroorganismus ein Mikroorganismus gemäß einem der Ansprüche 1 bis 4 ist und der zweite Mikroorganismus für die Synthese der PLA aus Milchsäure-Monomeren gentechnisch verändert ist.

## Claims

1. Recombinant microorganism genetically modified for
i) Synthesizing a lactic acid monomer by fermenting a carbon source;
ii) Depolymerizing polylactic acid (PLA)
wherein said microorganism expresses at least one enzyme for depolymerizing PLA, selected from a proteinase K, a lipase and a PLA depolymerase and a lactate dehydrogenase for synthesizing lactic acid.

2. Microorganism according to claim 1, genetically modified for
iii) Mitigating the degradation pathway of lactic acid monomer that it is able to synthesize.

3. Microorganism according to claim 1 or 2, genetically modified for
iv) Synthesizing PLA constituted of lactic acid monomer that it is able to synthesize.

4. Microorganism according to one of claims 1-3, wherein the microorganism is a lactic microorganism, preferably a lactic bacterium.

5. Process for producing monomer of lactic acid comprising the step of
- Contacting a microorganism according to one of claims 1-4 with a carbon source and with PLA that can be depolymerized by said microorganism.

6. Process for producing hydrocarbon monomer according to claim 5, further comprising the step of
- Recovering the lactic acid monomer produced.

7. Coculture of microorganisms comprising at least a first microorganism according to one of claims 1-4 and at least a second microorganism, optionally genetically modified, able to synthesize a polymer constituted of at least lactic acid monomers produced by the first microorganism.

8. Process for synthesizing polymers using a coculture of microorganisms according to claim 7, comprising the step of
- Contacting the coculture of microorganisms with a carbon source and a polymer that can be degraded by the first microorganism.

9. Process for synthesizing polymers using a coculture of microorganisms according to claim 8, further comprising the step of
- Recovering PLA produced by the second microorganism.

10. Process for synthesizing according to claim 9, for producing PLA, wherein the first microorganism is a microorganism according to one of claims 1-4, and the second microorganism is genetically modified for synthesizing PLA with lactic acid monomers.


### SEQUENCE LISTING

<110> Carbios
<120> Microorganisme recombinant
<130> B1321PC00
<160> 7
<170> PatentIn version 3.5
<210> 1
   <211> 717
   <212> DNA
   <213> Amycolatopsis sp. K104-1
<400> 1
   gtgaaattcg gcaagttcgt cctgctggcc gcgagcaccg cactggccgt cgtcggcctc      60
   ggcggtccgg cggccgccga cagcaccccg caggcccagc cgtcgatcat cggtggcagc     120
   aacgccacca gtggcccctg ggcggcccgg ctgttcgtca acggccggca gaactgcacc     180
   gcgacgatca tcgccccgca gtacatcctc accgccaagc actgcgtcag cagctccggc     240
   acctacacgt tccgcatcgg cagcctggac cagacgagcg gcggcacgat ggccaccggc     300
   tccacgatca cgcgctaccc gggctccgcc gacctggcga tcgtccggct caccacctcg     360
   gtgaacgcca cctactcgcc actcggcagc gtcggtgacg tttcggtcgg ccagaacgtc     420
   tcggtctacg gctggggcgc gaccagccag tgcggctccg agatcaactg ccagtcgcgg     480
   tacctgaagg tcgcgacggt gcgggtgaac tcgatcagct gcagcgacta caccggcggc     540
   gtcgccgtgt gcgcgaaccg cgtcaacggc atcaccgccg gcggcgactc cggcggcccg     600
   atgttcgctt ccggccgcca ggtcggcgtc gcgtcgacca gcgaccgggt gaacaacacg     660
   gcgtacacca acatcacgcg ttatcgcagc tggatttcgc aggtggcggg cgtctga        717
<210> 2
   <211> 1881
   <212> DNA
   <213> Cryptococcus sp. S-2
<220>
   <221> misc_feature
   <222> (1004)..(1594)
<220>
   <221> misc_feature
   <222> (1642)..(1770)
<400> 2
   gctggcctcg gcgtagcaaa ggcgatgcgg acgatccacg gcgaggccta ccagtccggc      60
   caggcctgtg atctgacgta tcggtgagtt gctggacgat cgtataccgg cactgagctg     120
   acggcgtcta gcgctcccgg agacgccatc gactacgcct acggcgtcac tgacatccgg     180
   tggtcgtatt ccgccgaact gcgagatacc ggcacagtga gttgggctgt gacgcctgca      240
   tacccctaca gaagctgatc cgcggtcagt acggcttcat gcttcccccg tcattaatcc      300
   gatcggctgc ggaggagacc acggccggac tgatgtacct gggcaaattc atctacaacc      360
   tcgagatagc cggctaagcc gccaatgatg ggcccggaag gcacctgtcc tcgtgtgacg      420
   ctagtgatga aaatgtaccg atgtatttta cttattctac cgcgatgagg agctccgttc      480
   gatccagact atcggtacag tcctgtcatg tgatgagact gcggttagtg ccggtgaata      540
   tgccaggtcc cacttcgcgc ggtactagtt acagtagtag tctggactct gccgagctcg      600
   cttaacagca acattactgg gcgtcgtgcc tgtgcacctg atcctgttta gcgtcttccg      660
   gttatgtcgg gatgaagtcg ttctcggacc catttttgaa cgaatggcgc cagagctgag      720
   gcgtggactc ggagcatatg caagatgtag gaggccatct gactgttgat tgatcggggg      780
   acgggggaat ggcctgccga gagaaacaca ggtctagcgg tgattgggac gagcggtggc      840
   gtgagactag cctggaatat cgggtgaatc aggtcctgac ttcgccgggt ggcaggatag      900
   caggtgccgg cttatcagat ataagaaggg gtcatgcccc aataccagcg acgacccact      960
   cacctctccg agccataccg cacaccccca gctcccgtca gacatgctcg tctccgctct     1020
   cgctctcgcg gtgctgtccg ctgcttctct cggccgagcc gcaccaacgc ccgagtccgc     1080
   cgaggcgcac gagctcgagg cccgcgccac gtccagcgct tgtccgcagt acgtcctgat     1140
   caacacgcga ggcacgggcg agccgcaagg ccagtcggcc ggcttccgaa cgatgaacag     1200
   ccagatcacc gccgcgctgt cgggtggcac catctacaac actgtctaca ccgccgattt     1260
   cagccagaac agcgcggccg gcacggccga catcatccgc cggatcaact cgggtctcgc     1320
   ggccaacccg aacgtgtgct acatcctcca agggtacagc cagggcgcgg ctgctaccgt     1380
   cgtcgcgctg caacagctcg gcacgagtgg agcggcgttc aacgccgtca agggtgtgtt     1440
   cctcattggc aacccggacc acaagtcggg cctgacttgc aacgtcgact cgaacggcgg     1500
   cactaccaca cgcaatgtca acggcctgtc ggtcgcgtac cagggctcgg tcccctcagg     1560
   atgggtcagc aagactctcg atgtctgcgc ttatgtacgt gcacgctgct catgatttga     1620
   ggacaagcgc tgacccggta gggcgacggc gtgtgcgaca ccgcgcacgg attcggtatc     1680
   aacgcacagc acctgtcgta ccctagtgac caaggcgtcc agaccatggg atacaagttt     1740
   gccgtcaaca agcttggcgg gtcggcctaa acggcactct tcgacctcga ttagttcaga     1800
   gactgaggca gttgtgatcg ctccggctgc tagggagtgg accagtatca cgtttacata     1860
   tgcacattat atctcgctag c                                                1881
<210> 3
   <211> 239
   <212> PRT
   <213> Cryptococcus sp. S-2
<400> 3
   Met Leu Val Ser Ala Leu Ala Leu Ala Val Leu Ser Ala Ala Ser Leu
   1               5                   10                  15
   Gly Arg Ala Ala Pro Thr Pro Glu Ser Ala Glu Ala His Glu Leu Glu
               20                  25                  30
   Ala Arg Ala Thr Ser Ser Ala Cys Pro Gln Tyr Val Leu Ile Asn Thr
           35                  40                  45
   Arg Gly Thr Gly Glu Pro Gln Gly Gln Ser Ala Gly Phe Arg Thr Met
       50                  55                  60
   Asn Ser Gln Ile Thr Ala Ala Leu Ser Gly Gly Thr Ile Tyr Asn Thr
   65                  70                  75                  80
   Val Tyr Thr Ala Asp Phe Ser Gln Asn Ser Ala Ala Gly Thr Ala Asp
                   85                  90                  95
   Ile Ile Arg Arg Ile Asn Ser Gly Leu Ala Ala Asn Pro Asn Val Cys
               100                 105                 110
   Tyr Ile Leu Gln Gly Tyr Ser Gln Gly Ala Ala Ala Thr Val Val Ala
           115                 120                 125
   Leu Gln Gln Leu Gly Thr Ser Gly Ala Ala Phe Asn Ala Val Lys Gly
       130                 135                 140
   Val Phe Leu Ile Gly Asn Pro Asp His Lys Ser Gly Leu Thr Cys Asn
   145                 150                 155                 160
   Val Asp Ser Asn Gly Gly Thr Thr Thr Arg Asn Val Asn Gly Leu Ser
                   165                 170                 175
   Val Ala Tyr Gln Gly Ser Val Pro Ser Gly Trp Val Ser Lys Thr Leu
               180                 185                 190
   Asp Val Cys Ala Tyr Gly Asp Gly Val Cys Asp Thr Ala His Gly Phe
           195                 200                 205
   Gly Ile Asn Ala Gln His Leu Ser Tyr Pro Ser Asp Gln Gly Val Gln
       210                 215                 220
   Thr Met Gly Tyr Lys Phe Ala Val Asn Lys Leu Gly Gly Ser Ala
   225                 230                 235
<210> 4
   <211> 4451
   <212> DNA
   <213> Thermobifida fusca
<220>
   <221> CDS
   <222> (2027)..(2932)
<400> 4
   acgtgtcctc tgcactgcaa tccccgcttc ccagcgcatc accggccccg gcgcgggccg      60
   gacgcggcgg ggtgctcccc gcgcccggct gggttgtggg tgcggtctgc acggtgttcg     120
   ccgtcgcggg cctcaccctg gtcccgccgt ggctgggcct gggatgggac gaggtcgtct     180
   acgtcagcca gtacgatccc cgcaatcccg ccgcgttctt cagcgcgccc cggtcgcgcg     240
   gggtgtcgct gctggccgcg ccggtggtgc tcgtcaccga ctcggtggtg gcgcttcggg     300
   tgtggctggc tgcggccgcg gccgtggcca tgggcgcggc gttctggccg tggctgcggc     360
   tgtatccgcg cagcggggtg gtgccgctcg cagccttcgg gtatgcgagc ctgtgggtca     420
   gcttgttcta cgcggccgcg gcgatgccca accatttcac ggcgatggcc gcggtgggcg     480
   cggtcggctg gttcctggtc gcggtccgcg aacccgcgtc ccggtctgcg ctggccgggc     540
   ttgcagccat gctggcggtg gccgggctga tgcggcccag tgacgcgttc tggttgaccg     600
   cgcctttggg gctggccgga cttgtcgtgc cgtcgtggcg gcgggtaccg ctgctcgcgg     660
   ccgtggccgg cggcggcctg gccggtgtgg caccgtggct ggtggaagcg gagctctcct     720
   acgggggcgt gctctcccgg ctggctcggg cctccgagat ccagggcggg accgggtgga     780
   ccctggctgt ggggtacgtg gtcaccgcgt tggacgggcc gctgctgtgc cgcccctgca     840
   ccgaggacct ggtgcgctgg cctgccctgc tctggccgat cgctttggtc gtactggtgg     900
   tgggcgggat tgtcggcgct caccgggcgg gacgtcccgc tctggggtgg ctgcctgtgg     960
   tggtcgccgg gtcgctcgcg ttcacctacc tgttcctcgt ctcctacacg gcgccgcgtt    1020
   tcctccagcc cacgtacgcg ctgctcatgc tgcccgctgc ggcaggactg cacgatgcgt    1080
   ggacggcgac ccggccccgg ctgcgtcccg tgctgggtgc cgggttggcc gtggcgatcc    1140
   tcggacatct cctcatccag ggggcgatcc tcacccactg ggtcaccgtc cacacggcgg    1200
   cccgggagaa ctatgcccgg ctcgccgagg agctgcatgc ggccgggcta cgcccgccgt    1260
   gcgtgctcac cggggacgag gccattccca tcgcctacta cgcggggtgc gcctcggcgg    1320
   cggcgtccgg caacaacacc acgcacaccc tggaggagct gctcgcggtg agccggaccg    1380
   taccgttcgg gctgctcgcc aaggaggacg gtccgcccga gtgggccgct gactgggacg    1440
   cgctgcccgt gggtcccgcc gacgacccgt ggtcgtgggt ggtgtacctc ccgccgtgga    1500
   gcccgctgtc cattccggaa tgagtccgag cgggtattct cgctacctat ttcagccccg    1560
   gagtcaggat tccgggcttt ttctctgtcc caccccaccc ccacatttat ggacatttcc    1620
   tcgcaaaaca cactatttga cctgtggttt ggcgagacac tggtgatttc acggatgcca    1680
   tccggctccc ccatgccgaa tagtgacgtt gcggttaaga cacagaaccg gttaccgccg    1740
   gatctcctta ccgcaacgtt gtgagcggcc taccgcaatg gctgaccacg acgaggcaga    1800
   ccctcgccca ccagtgcctg ccgcatcggc cccccgctgc gacggtcacg cccggcttcg    1860
   gactctcggg gacggcgccc cggtgggcat ggaccgttca gtgtccccac ggtgaacggc    1920
   ccaccatccc ccgcacatcc ggtctgcccc taccgtggcc agtgccgctc ctccctccgt    1980
   ccacgggcga ccctccgctt tcgcccttac acgaagagga tgtgca atg gct gtg       2035
                                                      Met Ala Val
                                                      1
   atg acc ccc cgc cgg gag cgc tct tcc ctg ctc tcc cga gct ctg caa      2083
   Met Thr Pro Arg Arg Glu Arg Ser Ser Leu Leu Ser Arg Ala Leu Gln
       5                   10                  15
   gtg acg gct gcg gct gcc aca gcg ctt gtg acc gcg gtc agc ctg gcc      2131
   Val Thr Ala Ala Ala Ala Thr Ala Leu Val Thr Ala Val Ser Leu Ala
   20                  25                  30                  35
   gcc ccc gct cat gcc gcc aac ccc tac gag cgc ggc ccc aac ccg acc      2179
   Ala Pro Ala His Ala Ala Asn Pro Tyr Glu Arg Gly Pro Asn Pro Thr
                   40                  45                  50
   gac gcc ctg ctc gaa gcc agc agc ggc ccc ttc tcc gtc agc gag gag      2227
   Asp Ala Leu Leu Glu Ala Ser Ser Gly Pro Phe Ser Val Ser Glu Glu
               55                  60                  65
   aac gtc tcc cgg ttg agc gcc agc ggc ttc ggc ggc ggc acc atc tac      2275
   Asn Val Ser Arg Leu Ser Ala Ser Gly Phe Gly Gly Gly Thr Ile Tyr
           70                  75                  80
   tac ccg cgg gag aac aac acc tac ggt gcg gtg gcg atc tcc ccc ggc      2323
   Tyr Pro Arg Glu Asn Asn Thr Tyr Gly Ala Val Ala Ile Ser Pro Gly
       85                  90                  95
   tac acc ggc act gag gct tcc atc gcc tgg ctg ggc gag cgc atc gcc      2371
   Tyr Thr Gly Thr Glu Ala Ser Ile Ala Trp Leu Gly Glu Arg Ile Ala
   100                 105                 110                 115
   tcc cac ggc ttc gtc gtc atc acc atc gac acc atc acc acc ctc gac      2419
   Ser His Gly Phe Val Val Ile Thr Ile Asp Thr Ile Thr Thr Leu Asp
                   120                 125                 130
   cag ccg gac agc cgg gca gag cag ctc aac gcc gcg ctg aac cac atg      2467
   Gln Pro Asp Ser Arg Ala Glu Gln Leu Asn Ala Ala Leu Asn His Met
               135                 140                 145
   atc aac cgg gcg tcc tcc acg gtg cgc agc cgg atc gac agc agc cga      2515
   Ile Asn Arg Ala Ser Ser Thr Val Arg Ser Arg Ile Asp Ser Ser Arg
           150                 155                 160
   ctg gcg gtc atg ggc cac tcc atg ggc ggc ggc ggc acc ctg cgt ctg      2563
   Leu Ala Val Met Gly His Ser Met Gly Gly Gly Gly Thr Leu Arg Leu
       165                 170                 175
   gcc tcc cag cgt ccc gac ctg aag gcc gcc atc ccg ctc acc ccg tgg      2611
   Ala Ser Gln Arg Pro Asp Leu Lys Ala Ala Ile Pro Leu Thr Pro Trp
   180                 185                 190                 195
   cac ctc aac aag aac tgg agc agc gtc acc gtg ccg acg ctg atc atc      2659
   His Leu Asn Lys Asn Trp Ser Ser Val Thr Val Pro Thr Leu Ile Ile
                   200                 205                 210
   ggg gcc gac ctc gac aca atc gcg ccg gtc gcc acg cac gcg aaa ccg      2707
   Gly Ala Asp Leu Asp Thr Ile Ala Pro Val Ala Thr His Ala Lys Pro
               215                 220                 225
   ttc tac aac agc ctg ccg agc tcc atc agc aag gcc tac ctg gag ctg      2755
   Phe Tyr Asn Ser Leu Pro Ser Ser Ile Ser Lys Ala Tyr Leu Glu Leu
           230                 235                 240
   gac ggc gca acc cac ttc gcc ccg aac atc ccc aac aag atc atc ggc      2803
   Asp Gly Ala Thr His Phe Ala Pro Asn Ile Pro Asn Lys Ile Ile Gly
       245                 250                 255
   aag tac agc gtc gcc tgg ctc aag cgg ttc gtc gac aac gac acc cgc      2851
   Lys Tyr Ser Val Ala Trp Leu Lys Arg Phe Val Asp Asn Asp Thr Arg
   260                 265                 270                 275
   tac acc cag ttc ctc tgc ccc gga ccg cgc gac gga ctc ttc ggc gag      2899
   Tyr Thr Gln Phe Leu Cys Pro Gly Pro Arg Asp Gly Leu Phe Gly Glu
                   280                 285                 290
   gtc gaa gag tac cgc tcc acc tgc ccg ttc tag gaagagaaca cgacgagtct    2952
   Val Glu Glu Tyr Arg Ser Thr Cys Pro Phe
               295                 300
   ttcctcccca ttctttcggt ggcggtcact gcggtggccg ccaccggccg ttttgtcccc    3012
   ccttttcatt cgaaaaatgc gacaaaccac cctttttgcc ccatcgcacc cccgataccg   3072
   aacgaagttc gggtacaaca ctggtggttt tacggatgca tgatcactgt gacttgcccc    3132
   atagtggcaa cgcagtcgag ataaggagcg cataaacccc aaacctcctt acctccgcct    3192
   gctgagcggc tcgttgaccg cacgtggccg ggcacaccgg cttcgcctac cggtcgcacg    3252
   cgccgtgcct tctccacccc cgcggcggaa aggcgcggcg ctcgcgggct ggaccgttcg    3312
   gaccccacgc gaacggcccg gaacccatgg cacccccgcg tccgggaggc aagcgccgcg    3372
   tgcctaccgc caacggcgcc gctcacctcc agtggcgagg cgggagtccg ggtccacgtc    3432
   catgcatgcc cccgcatgcg gcgcggcccg gccctgcaca gaaccgaaga ggacgtgcaa    3492
   tggctgtgat gaccccccgc cgggagcgct cttccctgct ctcccgggca ctgcgcttca    3552
   ccgccgcggc tgccacagcg cttgtgaccg cggtcagcct ggccgccccc gctcatgccg    3612
   ccaaccccta cgagcgcggc cccaacccga ccgacgccct gctcgaagcc cgcagcggcc    3672
   ccttctccgt gagtgaagaa cgggcctccc gcttcggtgc tgacggtttc ggcggcggca    3732
   ccatctacta cccgcgggag aacaacacct acggtgccgt ggcgatctcc cccggctaca    3792
   ccggcaccca ggcctctgtc gcctggctgg gcaagcgcat cgcctcccac ggcttcgtcg    3852
   tcatcaccat cgacaccaac accaccctcg accagccgga cagccgggcc cgccagctca    3912
   acgccgcgct ggactacatg atcaacgacg cctcgtccgc ggtgcgcagc cggatcgaca 3972
   gcagccgact ggcggtcatg ggccactcca tgggcggcgg cggcagcctg cgtctggcct 4032
   cccagcgtcc cgacctgaag gccgccatcc cgctcacccc gtggcacctc aacaagaact 4092
   ggagcagtgt gcgggttccc accctcatca tcggtgctga cctggacacc atcgctccgg 4152
   tcctcaccca cgcccggccc ttctacaaca gcctcccgac ctcgatcagc aaggcctacc 4212
   tggagctgga cggcgcaacc cacttcgccc cgaacatccc caacaagatc atcggcaagt 4272
   acagcgtcgc ctggctcaag cggttcgtcg acaacgacac ccgctacacc cagttcctct 4332
   gccccggacc gcgcgacgga ctcttcggcg aggtcgaaga gtaccgctcc acctgcccct 4392
   tctaggcggt agggtcccgc agcgagtcag caagatctcc ttcccggtgg ttgatactg 4451
<210> 5
   <211> 301
   <212> PRT
   <213> Thermobifida fusca
<400> 5
   Met Ala Val Met Thr Pro Arg Arg Glu Arg Ser Ser Leu Leu Ser Arg
   1 5 10 15
   Ala Leu Gln Val Thr Ala Ala Ala Ala Thr Ala Leu Val Thr Ala Val
   20 25 30
   Ser Leu Ala Ala Pro Ala His Ala Ala Asn Pro Tyr Glu Arg Gly Pro
   35 40 45
   Asn Pro Thr Asp Ala Leu Leu Glu Ala Ser Ser Gly Pro Phe Ser Val
   50 55 60
   Ser Glu Glu Asn Val Ser Arg Leu Ser Ala Ser Gly Phe Gly Gly Gly
   65 70 75 80
   Thr Ile Tyr Tyr Pro Arg Glu Asn Asn Thr Tyr Gly Ala Val Ala Ile
   85 90 95
   Ser Pro Gly Tyr Thr Gly Thr Glu Ala Ser Ile Ala Trp Leu Gly Glu
   100 105 110
   Arg Ile Ala Ser His Gly Phe Val Val Ile Thr Ile Asp Thr Ile Thr
   115 120 125
   Thr Leu Asp Gln Pro Asp Ser Arg Ala Glu Gln Leu Asn Ala Ala Leu
   130 135 140
   Asn His Met Ile Asn Arg Ala Ser Ser Thr Val Arg Ser Arg Ile Asp
   145                 150                 155                 160
   Ser Ser Arg Leu Ala Val Met Gly His Ser Met Gly Gly Gly Gly Thr
                   165                 170                 175
   Leu Arg Leu Ala Ser Gln Arg Pro Asp Leu Lys Ala Ala Ile Pro Leu
               180                 185                 190
   Thr Pro Trp His Leu Asn Lys Asn Trp Ser Ser Val Thr Val Pro Thr
           195                 200                 205
   Leu Ile Ile Gly Ala Asp Leu Asp Thr Ile Ala Pro Val Ala Thr His
       210                 215                 220
   Ala Lys Pro Phe Tyr Asn Ser Leu Pro Ser Ser Ile Ser Lys Ala Tyr
   225                 230                 235                 240
   Leu Glu Leu Asp Gly Ala Thr His Phe Ala Pro Asn Ile Pro Asn Lys
                   245                 250                 255
   Ile Ile Gly Lys Tyr Ser Val Ala Trp Leu Lys Arg Phe Val Asp Asn
               260                 265                 270
   Asp Thr Arg Tyr Thr Gln Phe Leu Cys Pro Gly Pro Arg Asp Gly Leu
           275                 280                 285
   Phe Gly Glu Val Glu Glu Tyr Arg Ser Thr Cys Pro Phe
       290                 295                 300
<210> 6
   <211> 31
   <212> DNA
   <213> artificial sequence
<220>
   <223> Amorce sens_site de restriction BamHI
<400> 6
   gatcggatcc gccacgtcca gcgcttgtcc g 31
<210> 7
   <211> 34
   <212> DNA
   <213> artificial sequence
<220>
   <223> Amorce anti-sens_site de restriction XhoI
<400> 7
   ccggaactcg agggccgacc cgccaagctt gttg 34